# EUROPEAN PATENT APPLICATION

(11) **EP 1 450 160 A1**
(43) Date of publication of application: **25.08.2004**
(21) Application number: 04003640.2
(22) Date of filing: 18.02.2004
(51) Int. Cl.: G01N 33/543

(54) **Surface plasmon resonance sensor for endotoxin**

(30) Priority: 21.02.2003 JP 2003043690
(71) Applicant: Nipro Corporation, Kita-ku, Osaka-shi, Osaka-fu, 531-8510 (JP)
(72) Inventor: Wada, Masaaki, c/o Nipro Corporation, Osaka-shi, Osaka 531-8510 (JP); Suetake, Toshinori, c/o Nipro Corporation, Osaka-shi, Osaka 531-8510 (JP)
(74) Representative: Forstmeyer, Dietmar, Dr. rer. nat., Dipl.-Chem.

(57) **Abstract**

A novel method for measuring endotoxin (ET) that can replace a Limulus test utilizes surface plasmon resonance (SPR) to facilitate the measurement of ET by automation without using expensive reagents. The method uses a sensor in which a substance, such as polymyxin B (PMX), that is capable of specifically adsorbing the ET and is immobilized on a SPR carrier. ET in a test specimen can be trapped by the substance, and thereby measured very easily and rapidly. In addition, the used sensor is reusable after treated with a regenerant.

## Description

This application claims a priority from Japanese Patent Application No. 2003-043690, which is incorporated herein by reference.

### BACKGROUND OF THE INVENTION

### 1. Field of the Invention

The present invention relates to a sensor for use in measuring endotoxin (hereinafter referred to as "ET") and to a method for measuring ET with the sensor. The present invention also relates to a method for evaluating test specimens on the basis of the measured ET values and to a method for reusing the sensor used. In particular, the measurement of ET according to the present invention utilizes a surface plasmon resonance (hereinafter referred to as "SPR") method.

### 2. Description of the Related Art

ET is a component of a bacterial body and is a polymolecular complex of lipopolysaccharide that is released from Escherichia coli, Vibrio cholerae, Pseudomonas aeruginosa, a salmonella, a dysentery bacillus, or the like when the body collapses. Although ET has relatively low toxicity, it is strongly pyrogenic and heat-resistant, and has destructive action on tumor cells.

Even a slight contamination by ET of pharmaceuticals or medical devices that directly come into contact with blood may cause serious results. Therefore, the level of contamination by ET must be strictly controlled; The United States Pharmacopeia and Japanese Pharmacopeia list ET tests. On the other hand, efforts to measure ET in blood are made for an early diagnosis of septicemia caused by Gram-negative bacterium infection, evaluation of therapeutic effects on Gram-negative bacteria infections, and prognostication.

As described above, ET receives attention in various applications. Different methods have been proposed for measuring ET.

Examples of conventional methods for measuring ET include a rabbit pyrogen test, a lethal test in chick embryo, radioimmunoassay, gas chromatography/mass spectrometry, and a Limulus test. Among them, the Limulus test is most sensitive, simple, and rapid, and is thus used in contamination tests for, for example, pharmaceuticals, water, and dialysates, clinical tests, or the like.

The method for measuring ET according to the Limulus test is based on the following principle: the inclusion of ET in a hemocyte extract (lysate) from a horseshoe crab will activate C-factor (ET sensitivity factor) and sequentially induce subsequent enzymatic chain reactions through individual paths, and a finally activated coagulase hydrolyzes a substrate. In general, the following two approaches, nephelometry and colorimetry, are employed: 1) In nephelometry, a coagulase that is finally activated through reactions between ET and C-factors in a lysate converts a coagulogen into a coagulin gel by its protease activity and the resulting change in turbidity during the gelation is measured with an optical analyzer. 2) In colorimetry, a chromophoric synthetic substrate in which p-nitroaniline (pNA) chromophore is bound to a synthetic peptide that has an amino acid sequence similar to that of a part of a coagulogen hydrolyzed during the conversion described above, is degraded by amidase activity of the activated coagulase, and the absorbance of the released pNA is measured.

However, since the conventional methods for measuring ET by the Limulus test are performed by manual methods, they cause undesirable variations in measured values between measurers, and disadvantageously require a new sensor in every measurement.

Thus, improved Limulus tests using, for example, a reagent containing β-1,3-glucanase (Japanese Unexamined Patent Application Publication No. 11-178599), a nephelometry time analysis method (Japanese Unexamined Patent Application Publication No. 8-211063), and absorbents of ET (Japanese Unexamined Patent Application Publication No. 2-193072) have been proposed. In addition, another method using SPR for measuring the interaction between ET and polymyxin B (PMX), which is known to antagonize the activity of ET, has also been published (FEBS Letters 445(1999) 420-424, J.B.C. 274(42) 29624-29627(1999)). However, this method using PMX does not disclose or suggest how to measure ET in a large amount of specimens rapidly and easily.

### SUMMARY OF THE INVENTION

It is an object of the present invention to provide a novel method for measuring ET that replaces the Limulus test. The method according to the present invention utilizes SPR to facilitate the measurement of ET by automation.

The present inventors accomplished the present invention by discovering that in the measurement of ET using SPR, by immobilizing a substance, such as PMX, capable of specifically adsorbing the ET onto a carrier to form a sensor, the ET in test specimens can be trapped by the substance, causing a change in the surface state of the sensor, and thereby can be measured very easily and rapidly. In addition, the used sensor is reusable after treated with a regenerant.

The present invention includes the following aspects:
1. A sensor for use in measuring ET by SPR according to the present invention comprises a substance that is capable of specifically binding to ET and is immobilized on a thin metal film carrier (hereinafter referred to as a ET sensor).
2. In this ET sensor, the substance capable of specifically binding to ET may be PMX or an anti-ET antibody.
3. In the ET sensor, the carrier may be a gold film.
4. A method for measuring ET by SPR after putting a test specimen into contact with an ET sensor that utilizes SPR and comprises a substance capable of specifically binding to the ET and immobilized on a thin metal film carrier.
5. In this method, the ET sensor may be put into contact with a test specimen and then allowed to react with an anti-ET antibody or PMX.
6. In the method, the ET sensor may be further allowed to react with another antibody that reacts with the anti-ET antibody.
7. In the method, the ET sensor may be further allowed to react with another antibody that reacts with PMX.
8. In the method described in Paragraph 6, another antibody is modified antibody thereof.
9. In the method described in Paragraph 7, another antibody is modified antibody thereof.
10. In the method, the ET may be a lipopolysaccharide (LPS), endotoxin, a pyrogenic substance, or a pyrogen.
11. In the method, the test specimen may be selected from a group consisting of a biological specimen, a culture solution, dialysate, waste of the dialysate, water for injection, a pharmaceutical agent, and pure water.
12. A method for evaluating ET contamination by the method described in Paragraph 10.
13. A method for diagnosing bacterial infections according to the method described in Paragraph 11.
14. A method for reusing a used ET sensor by putting the used ET sensor into contact with a regenerant that can elute ET trapped on the used ET sensor.
15. A method for manufacturing the ET sensor described in paragraph 3.
16. A kit for measuring ET comprises at least one of an ET sensor using SPR by immobilizing a substance that is capable of specifically binding to ET on a thin metal film carrier, a reagent for use in a method for measuring ET using SPR after putting the ET sensor into contact with a test specimen, and a reagent for use in a method for reusing described in paragraph 14.
17. Use of a SPR carrier chip (SPR sensor chip) for use in the method for measuring ET by SPR.

### BRIEF DESCRIPTION OF THE DRAWINGS

Fig. 1 shows the relation between the LPS concentration and the response when anti-ET was supplied in Experiment 2 and when anti-Mouse IgG was used in Experiment 3.

Fig. 2 shows the response as a function of the number of repeated measurements in Experiment 2 wherein a regenerant (12 mM NaOH + 0.05% Tween 20) was used after 10 ng/ml of LPS and anti-ET antibody were added.

### DESCRIPTION OF THE PREFERRED EMBODIMENTS

An ET sensor according to the present invention is prepared by immobilizing a substance, such as PMX, capable of specifically adsorbing ET onto a carrier for SPR.

The SPR method according to the present invention is particularly described in FEBS Letters 445(1999) 420-424. SPR carriers (SPR sensor chips) are also known and commercially available. The present invention utilizes a system based on the SPR technique and the SPR carrier. Currently, the system is advantageously available from, but not limited to, BIACORE as a biosensor. Various types of sensor chips are commercially available for SPR carriers. For example, a CM5 sensor chip is used in an example according to the present invention.

Preferred substances capable of specifically binding to ET include PMX and anti-ET antibody, but any substance capable of specifically binding to ET may be used. For example, nitrogen-containing heterocyclic compounds, such as histidine, histamine, and adenine may be used. PMX is commercially available and can be used with or without further purification. An anti-ET antibody, polyclonal or monoclonal, may be prepared according to any known method. In addition, commercially available anti-ET antibodies are also easily obtainable. For example, anti-ET antibodies may be obtained from Nanotools Antikorpertechnik in Germany or QED Bioscience, Inc. in U.S.A, and labeled anti-ET antibodies may be obtained from Biogenesis LTD. in G.B. While ET generally has many different names, the present invention includes LPS, endotoxin, lipopolysaccharides, a pyrogenic substance, and a pyrogen. The anti-ET antibody may be prepared by administering such ET to an animal together with a suitable adjuvant and, optionally, subsequently using technique for preparing a hybridoma.

The SPR carrier and the substance capable of specifically binding to ET are bound directly or via a spacer. The spacer includes, but not limited to, a monolayer film composed of a compound having a thiol group or a dithiol group, carboxymethyldextran, and streptavidin. Preferably, the amount of binding between the SPR carrier and the substance capable of specifically binding to ET is 1.0-2.5 ng/mm².

In the ET sensor according to the present invention, the substance capable of specifically binding to ET may be previously bound to the SPR carrier. Alternatively, in order to prepare the ET sensor before use, a kit composed of a substance capable of specifically binding to ET and a SPR carrier may be employed. Furthermore, only the SPR carrier may be provided for use in the method for measuring ET by SPR.

The test specimen in the measurement of ET with the sensor according to the present invention may be any object that may be contaminated by ET; for example, a biological specimen such as blood or urine, a culture solution, dialysate, waste of the dialysate, water for injection, a pharmaceutical agent, and pure water. While the measurement of ET in a lipid requires pretreatment with a surface-active agent or the like, a liquid test specimen is suitable for the measurement. Target ET, such as LPS, endotoxin, lipopolysaccharides, a pyrogenic substance, and pyrogen can essentially be detected as an equivalent. The sensor will be put into contact with the test specimen to selectively trap ET present in the test specimen. The trapped ET will be determined quantitative by the SPR measurement.

The sensor and 30-900 µl of the test specimen per sensor may be contacted for 2-60 min. Preferably, the sensor and 90-360 µl of the test specimen per sensor is contacted for 6-24 min.

For the measurement of a small amount of test specimen, we observed an improved sensitivity in SPR measurement when the trapped ET through the contact between the test specimen and the sensor was further reacted with the anti-ET antibody or PMX. The amount of the test specimen is about 50-300 µl (a solution containing the anti-ET antibody or PMX at a concentration of about 5-300 µg/ml or about 0.05-1 µg/ml, respectively).

A further increase in the sensitivity of the ET measurement can be achieved by reacting the test specimen, which has been reacted with the anti-ET antibody or PMX, with an antibody that can react with the anti-ET antibody or PMX, or with modified antibody thereof before the SPR measurement. The antibody that can react with the anti-ET antibody may be a commercially available anti-IgG antibody. The "modified antibody thereof" means a conjugated antibody that is modified by a polymer, such as protein (alkaline phosphatase, peroxidase, albumin, or the like) or dextran. The modification may be performed directly by a frequently-used method used for labeling antibodies, such as enzymes (Rinsyo Kensa Teiyo, Menekitekiteiryoho, Kanehara & Co. Ltd.) . The content of the anti-IgG antibody (about 5-300 µg/ml) is about 50-300 µl.

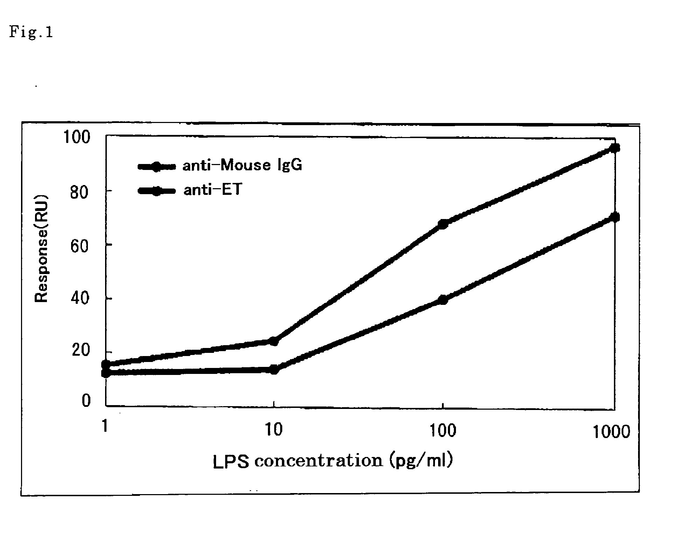

A calibration curve as shown in Fig. 1 can be obtained by the SPR measurement as a function of ET in the test specimen. This curve proves the significant effectiveness of the sensor and the method according to the present invention for quantitative analysis of ET.

Thus, the method according to the present invention can be extensively applied to the measurement of ET in contaminated test specimens. In particular, the method according to the present invention can be suitably applied to, for example, diagnosis of bacterial infections and determination of contamination by ET (a biological specimen such as blood or urine, a culture solution, dialysate, waste of the dialysate, water for injection, a pharmaceutical agent, pure water, and the like).

The present invention has another advantage in that the ET sensor can be reuse after regeneration. The "regeneration" means that ET trapped on the ET sensor from the test specimen is eluted from the ET sensor and thereby the ET sensor resumes the initial state. The regeneration is attainable by bringing the ET sensor into contact with a sufficient amount of regenerant described below. The ET sensor is reusable up to about ten times with desired sensitivity.

The regenerant may be an alkali solution, an acid solution, or a solution containing a surface-active agent. Examples of the alkali solution include aqueous solutions of NaOH, KOH, Na₂CO₃, NaHCO₃, NaOAc, and KOAc. Examples of the acid solution include aqueous solutions of HCl, AcOH, NH₄Cl, glycine, and citric acid. Examples of the surface-active agent include nonionic surface-active agents, such as polyoxyethylene sorbitan fatty acid esters or polyoxyethylene alkyl phenol ethers; bile acids, such as deoxycholic acid; anionic surface-active agents, such as sodium dodecyl sulfate. In addition, the alkali solution or the acid solution may contain the surface-active agent. Preferably, by way of example, 1-20 mM of the alkali solution contain 0.001-0.5% of the surface-active agent.

It is apparent from the description mentioned above that the present invention provides an ET sensor kit, for use in measuring ET or reusing the ET sensor, that includes the ET sensor and various reagents and/or solutions used in the measurement or in the reuse of the ET sensor.

The present invention will now be described in more detail with reference to, but are not limited to, the following example and experiments. In a method for measuring ET using SPR, as long as a substance capable of specifically adsorbing the ET is immobilized on a SPR carrier and used as a SPR sensor, various changes and modifications can be made in it without departing from the spirit and scope of the present invention.

### EXAMPLE 1

Preparation of ET sensor (ET sensor chip)
SPR was measured with a surface plasmon resonance detector (BIACORE), and a sensor chip CM5 (BIACORE) was used as an SPR carrier (sensor chip) of the present invention. HBS-EP (BIACORE) was used as a running buffer. The sensor chip was activated by feeding a solution containing N-ethyl-N'-(3-dimethylaminopropyl) carbodiimide hydrochloride (37.5 mg/ml) and N-hydroxy succinimide (5.75 mg/ml), which were surface activators of the sensor chip. Then, the sensor chip (1-1.2 mm²) was put into contact with 360 µl of aqueous acetic acid (pH 4.5) containing 10 mg/ml PMX for 24 min to immobilize PMX. Finally, 180 µl of 1 M ethanolamine-HCl aqueous solution (BIACORE) was added for blocking to form an ET sensor chip (ET sensor).

### Experiment 1 (Measurement of high level ET)

LPS (SIGMA) was used as ET to be measured. The ET sensor chip prepared in EXAMPLE 1 was used. A running buffer (pH 7.4) was prepared by filtering a solution containing 10 mM HEPES, 150 mM NaCl, and 3.4 mM EDTA to remove ET. A test solution was prepared by dissolving the LPS in this running buffer. The value observed by feeding 180 µl of the LPS solution for 12 min was proportional to the concentration of the LPS. The LPS of 10 ng/ml or larger was measurable.

### Experiment 2 (Measurement of low level ET)

In addition to the procedure of Experiment 1, after the addition of the LPS, 180 µl of an anti-ET antibody solution that was prepared by adjusting the concentration of an anti-ET antibody (nanoTools) at 10 µg/ml with the running buffer was added over 12 min. The observed value was proportional to the concentration of the LPS. The LPS of 10 pg/ml or larger was measurable (Fig. 1).

### Experiment 3 (Measurement of low level ET)

In addition to the procedure of Experiment 2, after the addition of the anti-ET antibody solution, 180 µl of a 100-fold diluted solution of a secondary antibody (anti-Mouse IgG AP conjugate (SIGMA)) was added over 12 min. The observed value was proportional to the concentration of the LPS. The LPS of 1 pg/ml or larger was measurable.

### Experiment 4 (Reuse)

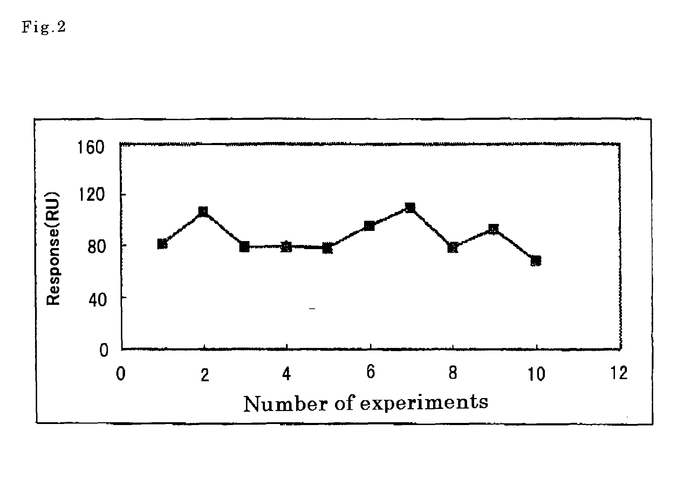

After the measurement of ET in Experiments 1-3, the experiments were able to repeat using the ET sensor chips (ET sensors) that were treated with a regenerant (for example, 12 mM NaOH + 0.05% Tween 20). Fig. 2 illustrates that, in Experiment 2, ET was repeatedly measurable after the addition of 10 ng/ml LPS and anti-ET antibody, by the treatment with the regenerant (12 mM NaOH + 0.05% Tween 20). Likewise, ET was repeatedly and consistently measurable for a regenerant containing 12 mM NaOH and 0.1% Triton X100, except that the value observed at the second measurement was half that at the first measurement.

## Claims

1. A sensor (ET sensor) for use in measuring endotoxin (ET) by a surface plasmon resonance (SPR), comprising a substance that is capable of specifically binding to ET and is immobilized on a thin metal film carrier.

2. A sensor according to Claim 1, wherein the substance is polymyxin B (PMX) or an anti-ET antibody.

3. A sensor according to Claim 1 or 2, wherein the carrier is a gold film.

4. A method for measuring ET by SPR after putting a test specimen into contact with an ET sensor that utilizes SPR wherein a substance capable of specifically binding to the ET is immobilized on a thin metal film carrier.

5. A method according to Claim 4, wherein the ET sensor is further allowed to react with an anti-ET antibody or PMX.

6. A method according to Claim 4, wherein the ET sensor is allowed to react with an anti-ET antibody and is then further allowed to react with another antibody that reacts with the anti-ET antibody.

7. A method according to Claim 4, wherein the ET sensor is allowed to react with PMX and is then further allowed to react with another antibody that reacts with PMX.

8. A method according to Claim 6, wherein another antibody is modified antibody thereof.

9. A method according to Claim 7, wherein another antibody is modified antibody thereof.

10. A method according to any one of Claims 4 to 9, wherein the ET is a lipopolysaccharide (LPS), endotoxin, a pyrogenic substance, or a pyrogen.

11. A method according to Claim 10, wherein the test specimen is selected from a group consisting of a biological specimen, a culture solution, dialysate, waste of the dialysate, water for injection, a pharmaceutical agent, and pure water.

12. A method for evaluating ET contamination by the method according to Claim 10.

13. A method for diagnosing a bacterial infection by the method according to Claim 11.

14. A method for reusing a used ET sensor by putting it into contact with a regenerant that can elute ET trapped on the used ET sensor, after the measurement by the method according to any one of Claims 4 to 9 is completed.

15. A method for manufacturing the ET sensor according to Claim 3.

16. A kit for measuring ET comprises at least one of an ET sensor using SPR by immobilizing a substance that is capable of specifically binding to ET on a thin metal film carrier, a reagent for use in a method for measuring ET using SPR after putting the ET sensor into contact with a test specimen, and a reagent for use in a method for reusing according to Claim 14.

17. Use of a SPR carrier chip (SPR sensor chip) for use in the method according to any one of Claims 4 to 9.
